# EUROPEAN PATENT APPLICATION

(11) **EP 2 110 071 A1**
(43) Date of publication of application: **21.10.2009**
(21) Application number: 09380077.9
(22) Date of filing: 16.04.2009
(51) Int. Cl.: A61B 3/11

(54) **Automatic system to obtain adjustment parameters for progressive lenses**

(30) Priority: 17.04.2008 ES 200801107
(71) Applicant: Tecnología Pro Informática, S.L., 46020 Valencia (ES); Asociación Industrial de Óptica, Color e Imagen - AIDO, 46980 Paterna (ES)
(72) Inventor: Ros Simo, Rafael A., 46020 Valencia (ES); Pascual Fortes, José Miguel, 46980 Paterna (Valencia) (ES); Esteve Taboada, Jose Juan, 46980 Paterna (valencia) (ES); Mico Serrano, Vicente, 46980 Paterna, Valencia (ES); Hervas Juan, Juan, 46980 Paterna, Valencia (ES); Simon Martin, Santiago, 46980 Paterna, Valencia (ES)
(74) Representative: Carvajal y Urquijo, Isabel

(57) **Abstract**

Automatic system to obtain adjustment parameters for frames with progressive lenses comprising, at least:
- a head (10) which also comprises:
- means for obtaining stereoscopic images,
- means for emitting a light source;
- a lens;

- means for the elevation (20) of the head (10), so that said head is adapted to the client's size;
- means for processing images (30);
- means for visualizing and interacting (40) with the system; and
- means to indicate the client's position (50);

wherein the means for processing images (30) are connected to the means for obtaining images and the visualization and interaction means (40), so that a plurality of optometric parameters can be captured and saved.

## Description

### OBJECT OF THE INVENTION

The object of the present invention is an automatic system to obtain adjustment parameters for frames with progressive lenses, a solution which can be applied to the field of optics-optometry, based on the use of a system for capturing stereoscopic images, image processing means and user interaction means, all of which in such a way that the parameters needed are obtained in a fast and simple manner.

### BACKGROUND OF THE INVENTION

No system or device with the characteristics of the system described below is known by the inventor, who is an expert on the subject.

### DESCRIPTION OF THE INVENTION

In order to solve the aforementioned problems, it is hereby presented an automatic system to obtain adjustment parameters for frames with progressive lenses, object of the present invention patent. Said system comprises a head with means for capturing stereoscopic images, processed by means specific to that end and visualized on a touchscreen which enables the user to interact with the system so that it works automatically and has the following functionalities, advantageous with respect to the current state of the art:
- Obtaining all the adjustment parameters needed to adapt the frames with progressive lenses, being said parameters:
   IPD: Interpupillary Distance.
   NPD: Nasal-pupillary Distance.
   Lower height: base-frame distance.
   Lenses diameters on the frame chosen by the patient.
   Pre-gauging function of the lenses:
      Width and height of the Box.
      Upper and lower distance.
      Nasal and temporal distance.
      Lenses diameters on the frame.
- Facilitating and personalizing the adjustment and adaptation of progressive lenses.
- Obtaining precise 3D measures by means of technology based on stereoscopic vision.
- Very easy to use, and fast to automatically obtain results.
- Stereoscopic measurement: more precision than with the manual ruler. Obtaining real 3D coordinates of the pupils, being robust with respect to patient's rotations.
- Automatic detection of pupil centers.
- Measurement taken under far vision conditions.

This new adjustment system enables to obtain a stereoscopic measure of the interpupillary distance, that is to say, it enables to obtain the coordinates of the pupils in the tridimensional space (3D), thus providing more precision than that obtained with the manual ruler. This fact also enables that the measurement of the interpupillary distance tolerates rotations of the patient's head.

Moreover, another difference with respect to traditional systems is that the pupil centers are automatically detected, as long as the contrast conditions are adequate. In the cases in which automatic detection is not feasible, the manual option is always available for the user to locate the pupils in the correct position. The automatic detection of pupil centers is carried out without using special supports on the frame, allowing for automatic detection when frames with demo lenses are used.

Another characteristic which distinguishes the optic system being claimed is that the interpupillary distance is measured under far vision conditions, since it uses as a fixing point a stimulus located in the optical infinity, using an optical system specially designed to that end.

The system requires minimum maintenance and enables to obtain the final results in a report format with real-scale images, so that the optician-optometrist can directly verify the measurements using the physical frame on the printed report.

To summarize, the design and functioning of the system herein described enables to eliminate great part of the manual operations that the optician-optometrist performs when adapting the lenses, thus avoiding repetitive errors that would otherwise distort the measurements obtained.

### BRIEF DESCRIPTION OF THE DRAWINGS

The following is a brief description of a series of drawings which will help understand the invention better and which clearly relates to an embodiment of said invention which is presented as a non-limiting example thereof.

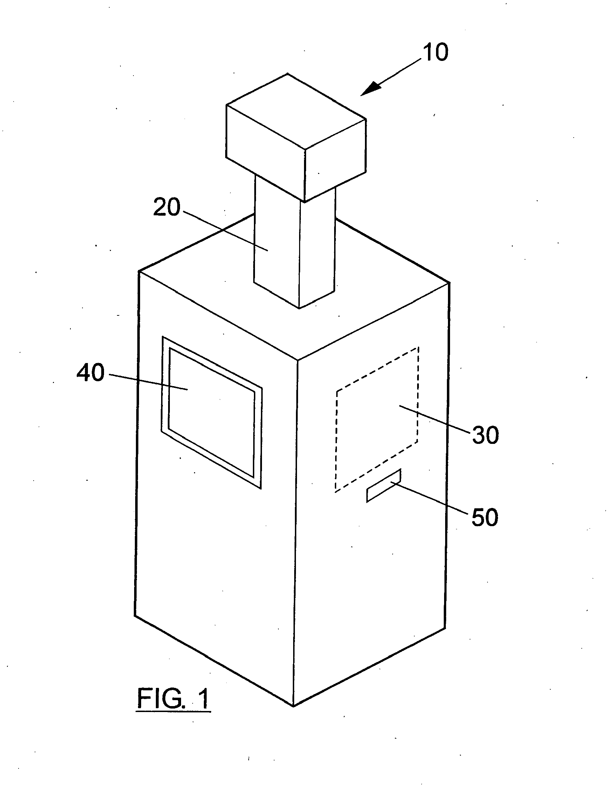

Figure 1 shows a perspective view of the automatic system to obtain adjustment parameters for frames with progressive lenses, object of the present invention.

### PREFERRED EMBODIMENT OF THE INVENTION

As it can be seen in the attached drawing, the automatic system to obtain adjustment parameters for frames with progressive lenses, object of the present invention, comprises at least:
- a head (10) which also comprises:
   - means for obtaining stereoscopic images, comprising, in turn, a high resolution camera, with 10 or more megapixels and optical means based on first surface mirrors
   - means for emitting a light source;
   - a lens, having 100mm diameter, suitable for binocular visualization of the light source emitted, where the work distance is adjusted to far vision conditions;
- means for the elevation (20) of the head (10), so that said head is adapted to the client's size;
- means for processing images (30);
- means for visualizing and interacting (40) with the system, preferably a touchscreen; and
- means to indicate the client's position (50);
wherein the means for processing images (30) are connected to the means for obtaining images and the visualization and interaction means (40), so that a plurality of optometric parameters can be captured and saved.

## Claims

1. Automatic system to obtain adjustment parameters for frames with progressive lenses **characterized in that** it comprises, at least:
- a head (10) which also comprises:
- means for obtaining stereoscopic images, comprising, in turn, a high resolution camera and optical means based on first surface mirrors;
- means for emitting a light source;
- a lens suitable for binocular visualization of the light source emitted, where the work distance is adjusted to far vision conditions;
- means for the elevation (20) of the head (10), so that said head is adapted to the client's size;
- means for processing images (30);
- means for visualizing and interacting (40) with the system; and
- means to indicate the client's position (50);
wherein the means for processing images (30) are connected to the means for obtaining images and the visualization and interaction means (40), so that a plurality of optometric parameters can be captured and saved.

2. System according to claim 1, **characterized in that** the resolution of the camera is 10 megapixels or more.

3. System according to claims 1 and 2, **characterized in that** the lens diameter is equal to or higher than 100mm.

4. System according to claims 1 to 3, **characterized in that** the visualization and interaction means (40) consist in a touchscreen.

5. System according to claims 1 to 4, **characterized in that** the means to indicate the client's position (50) consist in a laser pointer.
